(19) Europäisches Patentamt
European Patent Office
Office européen des brevets

(11) EP 0 876 144 B1

(12) **EUROPÄISCHE PATENTSCHRIFT**

(45) Veröffentlichungstag und Bekanntmachung des Hinweises auf die Patenterteilung:
**31.10.2001 Patentblatt 2001/44**

(21) Anmeldenummer: **97914084.5**

(22) Anmeldetag: **27.01.1997**

(51) Int Cl.[7]: **A61K 31/41**

(86) Internationale Anmeldenummer:
**PCT/DE97/00135**

(87) Internationale Veröffentlichungsnummer:
**WO 97/26968 (31.07.1997 Gazette 1997/33)**

(54) **THERAPEUTISCHE MITTEL, ENTHALTEND EBSELEN GEGEN ASTHMA**

THERAPEUTIC ANTI-ASTHMA AGENTS CONTAINING EBSELEN

AGENTS THERAPEUTIQUES CONTENANT EBSELEN, AGISSANT CONTRE L'ASTHME

(84) Benannte Vertragsstaaten:
**AT BE CH DE DK ES FI FR GB GR IE IT LI LU MC NL PT SE**

(30) Priorität: **26.01.1996 JP 1184896**

(43) Veröffentlichungstag der Anmeldung:
**11.11.1998 Patentblatt 1998/46**

(73) Patentinhaber: **A. Nattermann & Cie. GmbH
50829 Köln (DE)**

(72) Erfinder:
- **UCHIDA, Yoshiyuki
Tsukaba-shi, Ibaraki (JP)**
- **AKASHI, Akira
Tokyo (JP)**

(74) Vertreter: **Fischer, Hans-Jürgen et al
Aventis Pharma Deutschland GmbH,
Patent- und Lizenzabteilung,
Gebäude K 801
65926 Frankfurt am Main (DE)**

(56) Entgegenhaltungen:
**EP-A- 0 729 756** **DE-A- 19 506 669**
**GB-A- 2 238 310** **US-A- 4 778 814**

- **PATENT ABSTRACTS OF JAPAN vol. 096, no. 001, 31.Januar 1996 & JP 07 233056 A (DAI ICHI SEIYAKU CO LTD), 5.September 1995,**
- **BIOCHEM. PHARMACOL. (BCPCA6,00062952);94; VOL.48 (1); PP.65-74, HUMBOLDT UNIVERSITY BERLIN;INSTITUTE BIOCHEMISTRY; BERLIN; D-10115; GERMANY (DE), XP000654416 SCHEWE C ET AL: "Strong inhibition of mammalian lipoxygenases by the antiinflammatory seleno-organic compound ebselen in the absence of glutathione"**
- **AGENTS ACTIONS (AGACBH,00654299);88; VOL.24 (3-4); PP.313-19, KAROLINSKA INST.;DEP. TOXICOL.; STOCKHOLM; S-104 01; SWED. (SE), XP000654500 COTGREAVE I A ET AL: "The anti-inflammatory activity of Ebselen but not thiols in experimental alveolitis and bronchiolitis"**
- **DATABASE WPI Derwent Publications Ltd., London, GB; AN 95-353429 XP002032846 & IT 1 250 751 B (BOEHRINGER MANNHEIM) 24.April 1995**

EP 0 876 144 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**Beschreibung**

Die vorliegende Erfindung betrifft ein therapeutisches Mittel gegen Asthma, das wirksam asthmatische Reaktionen unterdrückt, insbesondere späte asthmatische Reaktionen beim Bronchialasthma.

Allgemein wurde erkannt, daß Bronchialasthma als eine Erkrankung gilt, die durch Kontraktion von weichen Muskeln im Luftweg aufgrund von Typ I Anaphylaxe verursacht wird. Jedoch haben kürzliche Erkenntnisse in diesem Forschungsgebiet aufgezeigt, daß ein Teil der asthmatischen Pathogenese dadurch verursacht wird, daß entgegengesetzte Luftbegrenzung, Luftwegentzündung, Mucus Hypersekretion und Neuausbildung der Luftwegstruktur in Folge der chronischen Entzündung ausgebildet werden. Von daher sollte eine pharmakologische Therapie über das Verständnis dieser Pathogenese hinaus dies entsprechend berücksichtigen.

Die zur Zeit verfügbaren Medikamente für Asthma sind Verringerungsmittel für die entgegengesetzte Luftbegrenzung, wie zum Beispiel Beta-Agonisten, und Kontrollmittel, so zum Beispiel Cortisoninhalationsmittel, zur Vermeidung von Symptomen durch Unterdrückung von Luftwegsentzündungen. Ein Beta-Agonist wirkt schnell in bezug auf eine Bronchialverengung und den damit verbundenen Begleitsymptomen, so zum Beispiel Husten, Brustenge und Keuchen. Ein inhaliertes Corticosteroid muß dauerhaft und täglich benutzt werden, um eine dauerhafte Kontrolle bei chronischem Asthma sicherzustellen.

Jedoch hat ein Beta-Agonist einige nachteilige Nebenwirkungen, wie zum Beispiel Stimulation der Herzgefäße, Muskelzuckungen und Hypokaliämie und Reizungen. Desweiteren verstärkt ein inhaliertes Cortisosteroid Candidiasis (oropharymgeal candidiasis), Dysphonie und gelegentliches Husten durch Reizung der oberen Luftwege, so daß das Risiko von systemischen Effekten eines Inhalationsmittels geringer ist als die systemischen Corticosteroide. Die lange Verwendung von oral oder parenteral applizierten Corticosteroiden kann ernsthafte Nebenwirkungen, wie zum Beispiel Osteoporose, Bluthochdruck, Diabetes, Hypophyseerkrankungen, grauer Star, Korpulenz, Muskelschwäche und Hautverdünnungen führen, die eine Hauterkrankung (cutaneous striae) und eine leichte Druckempfindlichkeit bewirken. Kontrollmittel werden für eine lange Zeit dargereicht und von daher sollten die systemischen Nebeneffekte von solchen Mitteln vermieden oder verringert werden.

Von dem Konzept, daß Kontrollmittel minimale Nebeneffekte mit Ausnahme in bezug auf die hauptsächlichen bronchialerweiternden oder entzündungshemmende Effekte besitzen, wurden anti-allergische Mittel, wie beispielsweise Histamin-Rezeptor-Antagonisten, Leukotrien-Rezeptor-Antagonisten, Thromboxan-synthetase-Inhibitoren/Rezeptor-Antagonisten entwickelt. Jedoch gibt es nicht ausreichende Daten bezüglich ihrer klinischen Vorteile in der Langzeitbehandlung von Asthma und von daher wird die Entwicklung von höher wirksamen entzündungshemmenden Mitteln und weiteren klinischen Erprobungen erwartet.

Es wird angenommen, daß die auftretenden späten asthmatischen Reaktionen nach Reizung des Immunsystems mit einer Luftwegsentzündung begleitet ist. Zwei Arten der asthmatischen Reaktionen wurden unmittelbar nach der Antigenreizung und einige Stunden danach beobachtet. Die frühere Reaktion wird als unmittelbare asthmatische Reizung (IAR) und das darauf folgende Phänomen als späte asthmatische Reizung (LAR) bezeichnet. Die unmittelbare asthmatische Reizung wurde durch die Luftwegsbegrenzung, die von der akuten Bronchialverengung herrührt, hervorgerufen durch den Allergenausstoß, erkannt, während die späte asthmatische Reizung eine Luftwegsentzündung im Luftweg begründet. Die Luftwegsentzündung, die üblicherweise durch eine extensive Infiltration von Cosinophilen, Zellen und von einzelligen Zellen gekennzeichnet ist, bewirkt eine Schwellung der Luftwegswände mit oder ohne leichte Muskelkontraktion. Derartige pathologische Änderungen werden nicht nur allein mit der späten asthmatischen Veränderung sondern auch mit der Luftwegshyperreaktivität und der Asthmaverschlimmerung in Verbindung gebracht (Metzger, W.J., Hunninghake, G.W. and Richardson, H.B.: Late Asthmatic Responses; Inquiry into Mechanism and Significance, Clin. Rev. Allergy 3:145, 1985). Der Mechanismus derartiger pathologischer Erscheinungen ist bis jetzt noch nicht vollständig erforscht.

Dementsprechend ist es erwünscht, ein Mittel zur Behandlung von Bronchialasthma zu entwickeln, das eine ausgezeichnete Wirksamkeit ähnlich zu der von Adrenal-Cortex-Hormonen besitzt, das gleichzeitig sowohl die unmittelbaren asthmatischen Reaktionen als auch die späten asthmatischen Reaktionen unterdrückt und das sicher ist.

Es ist bekannt, daß 2-Phenyl-1,2-Benzoisoselenazole-3(2H)-one, das üblicherweise auch als Ebselen bezeichnet wird und das eine typische in der vorliegenden Erfindung verwendete Verbindung darstellt, eine deutliche Lipoxygenase inhibierende Wirkung besitzt (Peter Kuhl et al, Prostaglandins, 31 (1986), 1029-1048). Jedoch ist niemals darüber berichtet worden, daß Ebselen bei der Behandlung von Asthma wirksam ist.

Unter den vorstehend genannten Umständen haben die Erfinder ausführliche Studien durchgeführt und festgestellt, daß Ebselen eine hohe Wirksamkeit in bezug auf die Unterdrückung von asthmatischen Reaktionen, insbesondere in bezug auf späte asthmatische Reaktionen, bei Bronchialasthma besitzen.

Dementsprechend stellt die vorliegende Erfindung ein therapeutisches Mittel zur Behandlung von Asthma zur Verfügung, das als Wirksubstanz Ebselen und/oder ein pharmazeutisch akzeptierbares Salz davon enthält.

Die Figur 1 zeigt die Wirksamkeit des Unterdrückungseffektes von Ebselen bei asthmatischen Reaktionen bei Meerschweinchen, die durch Antigene induziertes Asthma erkrankt sind.

Die vorliegende Erfindung umfasst die Verbindung 2-Phenyl-1,2-Benzoisoselenazole-3(2H)-one, das durch die folgende Formel wiedergegeben ist:

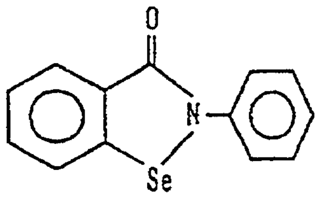

Darüber hinaus können in der vorliegenden Erfindung pharmazeutisch akzeptable Salze, die von Ebselen herstammen, verwendet werden.
Ebselen und pharmazeutisch akzeptable Salze davon besitzen exzellente Wirksamkeiten in bezug auf die Unterdrückung von unmittelbaren oder späten asthmatischen Reaktionen bei Bronchialasthma, insbesondere bei späten asthmatischen Reaktionen, wie dies in den nachstehend wiedergegebenen Ausführungsbeispielen belegt wird.
Darüber hinaus konnte gefunden werden, daß Ebselen sehr sicher ist, wie dies durch die nachfolgenden und in der Tabelle 1 wiedergegebenen $LD_{50}$-Werte bewiesen wird, wobei diese Werte das Ergebnis eines Toxizitätstests unter Verwendung von Mäusen und Ratten, denen diese Verbindungen oral oder intraperitoneal verabreicht wurden, darstellen. Selbst dann, wenn diese Verbindungen in hohen Dosen verabreicht werden, zeigen sie keine Nebeneffekte, die ernsthafte Probleme verursachen.

Tabelle 1

| Tier | Applikationsweg | $LD_{50}$ (mg/kg) |
|---|---|---|
| Maus | p.o. | > 6810 |
| | i.p. | 740 |
| Ratte | p.o. | > 6810 |
| | i.p. | 580 |

Die erfindungsgemäßen Mittel zur Behandlung von Asthma können in verschiedenen Formen von Zubereitungen für die orale oder parenterale Verwendung formuliert werden, so zum Beispiel als Tabletten, Kapseln, Puder, feine Granulate, Flüssigkeiten, Suspensionen, Emulsionen, Trockensirups, Inhalationsmittel, Injektionen und Zäpfchen. Derartige Zubereitungen werden nach den üblichen Methoden dadurch hergestellt, daß zu Ebselen oder zu einem pharmazeutisch akzeptierbaren Salz davon Gleitmittel, Sprengmittel, Bindemittel oder Trägermittel zugesetzt werden.
Die Dosis des Ebselens oder des pharmazeutisch akzeptierbaren Salzes davon hängt von dem Applikationsweg, dem Zustand des Patienten, dem Körpergewicht des Patienten o. dgl. ab. Im Falle der oralen Darreichung variiert die Dosis üblicherweise bei einem Erwachsenen zwischen 100 bis 2.000 mg/Tag, vorzugsweise zwischen 200 bis 1.000 mg/Tag.
Wie bereits vorstehend beschrieben, unterdrücken die erfindungsgemäßen Mittel sowohl die unmittelbaren als auch die späten asthmatischen Reaktionen bei Bronchialasthma. Insbesondere bewirken die Mittel herausragende Effekte in bezug auf die späten asthmatischen Reaktionen. Darüber hinaus besitzen die erfindungsgemäßen Mittel eine geringe Toxizität und sind von daher sicher in bezug auf Menschen.

**Testbeispiel**

Die Wirksamkeiten von Ebselen wurden unter Verwendung von Meerschweinchen, die ein Antigen induziertes Asthma besitzen, durch Überprüfung der Lungenfunktionen unter Bewußtsein und spontaner Atmung getestet.
Verfahren:

1. Tiere

Jedes der weiblichen Hartley Meerschweinchen (SLC) (4 Wochen alt, Körpergewicht etwa 250 bis 300 g) wurden vorbehandelt mit Cyclophosmid (2 mg/kg). Nach zwei Tagen wurden intraperitoneal Ovalbumin (OA, 1 mg)

und Aluminiumgel (1 g) injiziert. Drei Wochen später wurden wiederum OA (0,1 mg) und Aluminiumgel (1 g) intraperitoneal injiziert. Die so behandelten Meerschweinchen wurden als sensibilisierte Tiere benutzt. Zu diesem Testzeitpunkt waren die Meerschweinchen elf Wochen alt und hatten ein Körpergewicht von 450 bis 580 g.

2. Apparate und Werkzeuge

Körper-Pressmograph, Drucktyp

Pulmotacograph (TP-601G, Nippon Kodensha)
Differentialmeßwandler (T-601, Nippon Kodensha)
Gasflußwiderstandsrohr (Lilly type, Nippon Kodensha)
Oszilloskop (DS-9121, Iwasaki Tsushinki)
Computer (Macintosh Centris 660 AV, Apple)
Software (zur Analyse der Atmung basierend auf LabView für Macintosh 3.01)
Zerstäuber (NE-U10, Tateishi Denki)

3. Verfahren zur Darreichung der Mittel und Induzierung der Antigene

Gruppe von mit Dexamethason vorbehandelten Meerschweinchen
Dexamethason (1 mg/kg) wurde in 100 % DMSO (1 ml) gelöst. Diese Lösung wurde während vier aufeinander folgende Tage intraperitoneal injiziert. Die letzte intraperitoneale Injektion wurde 24 Stunden vor der Induzierung der Antigene durchgeführt.
Gruppe der mit Ebselen vorbehandelten Meerschweinchen Ebselen (5 mg/kg) wurde in 100 % DMSO (1 ml) gelöst. Diese Lösung wurde intraperitoneal 30 Minuten vor der Induzierung der Antigene injiziert.

Kontrollgruppe

100 % DMSO (1 ml) wurde intraperitoneal 30 Minuten vor der Induzierung der Antigene injiziert.

Verfahren zur Induzierung von Antigenen

OA (40 mg) wurde in einer physiologischen Salzlösung (4 mg/ml) gelöst. Diese Lösung wurde 2 Minuten lang unter Verwendung eines Ultraschallzerstäubers inhaliert.

4. Verfahren zur Überprüfung der Lungenfunktion

In Übereinstimmung mit dem Verfahren von Agrawal (Agrawal, K. P.; Specific Airway Conductance in Guinea Pigs: "Normal Values and Histamine Induced Fall", Respiratory Physiology 43:23, 1981) wurde jedes Meerschweinchen auf einem Körper-Pressmograph, Drucktyp, fixiert und die prozentuale Variation des spezifischen Luftweg-Leitwertes (sGaw) wurde gemessen. Der Luftfluß durch die Nase und die Änderung des Innendruckes der Box wurden aufgezeichnet. Die entsprechenden Wellenformen' wurden digital bei 1.024 Hz gesammelt und die Punkte vom Ende des Ausatmens bis zum Beginn des Einatmens wurden als rückläufige Linie aufgezeichnet. Unter Verwendung der Neigung (tan) der rückläufigen Linie wurde der sGaw erhalten. Der sGaw-Wert wurde vor der Induzierung der Antigene gemessen. Nachdem eine physiologische Salzlösung für zwei Minuten inhaliert wurde, wurde der sGaw nochmals gemessen, um sicherzustellen, daß sich keine Daten geändert hatten. Der zu diesem Zeitpunkt gemessene Wert wurde als 100 % angenommen und die prozentuale Variation des sGaw wurde nach Induzierung der Antigene bestimmt.
Die Ergebnisse sind in Figur 1 dargestellt. Bei der Gruppe von Meerschweinchen; die mit Ebselen vorbehandelt wurden, waren sowohl die unmittelbaren als auch die späten asthmatischen Reaktionen wirkungsvoller unterdrückt als bei den Meerschweinchen der Kontrollgruppe.
Wenn die mit Ebselen vorbehandelte Gruppe mit der mit Dexamethason vorbehandelten Gruppe verglichen wird, sind die Bereiche der Unterdrückung der unmittelbaren asthmatischen Reaktionen überwiegend gleich. Jedoch wurden bei den mit Ebselen vorbehandelten Gruppen die späten asthmatischen Reaktionen erheblich und vollständig unterdrückt, während bei der Kontrollgruppe die späten asthmatischen Reaktionen zwischen 180 und 420 Minuten beobachtet wurden.
In diesem Zusammenhang ist darauf hinzuweisen, daß die Dexamethason-Dosis von 1 mg/kg, die über 4 Tage den Meerschweinchen verabreicht wurde, ungefähr einer Dexamethason-Dosis für einen Menschen (Körpergewicht 60 kg) von 240 mg (60 mg x 4 Tage) entspricht. Diese Dexamethason-Dosis entspricht 2.400 mg von Prednisolon (0,5 mg Dexamethason ist äquivalent zu 5 mg Prednisolon).

Diese Menge von Prednisolon (2.400 mg) entspricht der Menge, die in einer Pulstherapie verbraucht wird, in der 1 g/Tag Prednisolon in Folge während 3 Tage dargereicht wird.

Die vorstehenden Ausführungen beweisen eindeutig, daß Ebselen eine höhere Wirksamkeit im Vergleich zu solchen Therapien besitzt, die in früheren klinischen Situation angewendet wurden.

**Beispiel 1**

Tabletten

| Unter Verwendung des bekannten Verfahrens wurden Tabletten der nachfolgenden Zusammensetzung hergestellt: | |
|---|---|
| Ebselen | 50 mg |
| Carboxymethylcellulose | 25 mg |
| Stärke | 5 mg |
| kristalline Cellulose | 40 mg |
| Magnesiumstearat | 2 mg |
| Summe | 122 mg |

Zusammenfassend ist festzuhalten, daß die vorliegende Erfindung ein sicheres therapeutisches Mittel zur Behandlung von Asthma, das sowohl die unmittelbaren als auch die späten asthmatischen Reaktionen bei Bronchialasthma unterdrückt, beschreibt.

## Patentansprüche

1. Verwendung von 2-Phenyl-1,2-Benzoisoselenazole-3(2H)-one oder ein pharmazeutisch akzeptables Salz davon zur Herstellung eines Arzneimittels zur Behandlung von Bronchialasthma.

2. Verwendung von 2-Phenyl-1,2-Benzoisoselenazole-3(2H)-one oder ein pharmazeutisch akzeptables Salz davon zur Herstellung eines Arzneimittels zur Unterdrückung der späten asthmatischen Reaktionen.

## Claims

1. The use of 2-phenyl-1,2-benzoisoselenazol-3(2H)-one or a pharmaceutically acceptable salt thereof for the production of a pharmaceutical for the treatment of bronchial asthma.

2. The use of 2-phenyl-1,2-benzoisoselenazol-3(2H)-one or a pharmaceutically acceptable salt thereof for the production of a pharmaceutical for the suppression of late asthmatic reactions.

## Revendications

1. Utilisation de la 2-phényl-1,2-benzoisosélénazol-3(2H)-one ou d'un sel pharmaceutiquement acceptable de celle-ci pour la production d'un médicament pour le traitement de l'asthme bronchique.

2. Utilisation de la 2-phényl-1,2-benzoisosélénazol-3(2H)-one ou d'un sel pharmaceutiquement acceptable de celle-ci pour la production d'un médicament pour réprimer les réactions asthmatiques tardives.

Fig 1

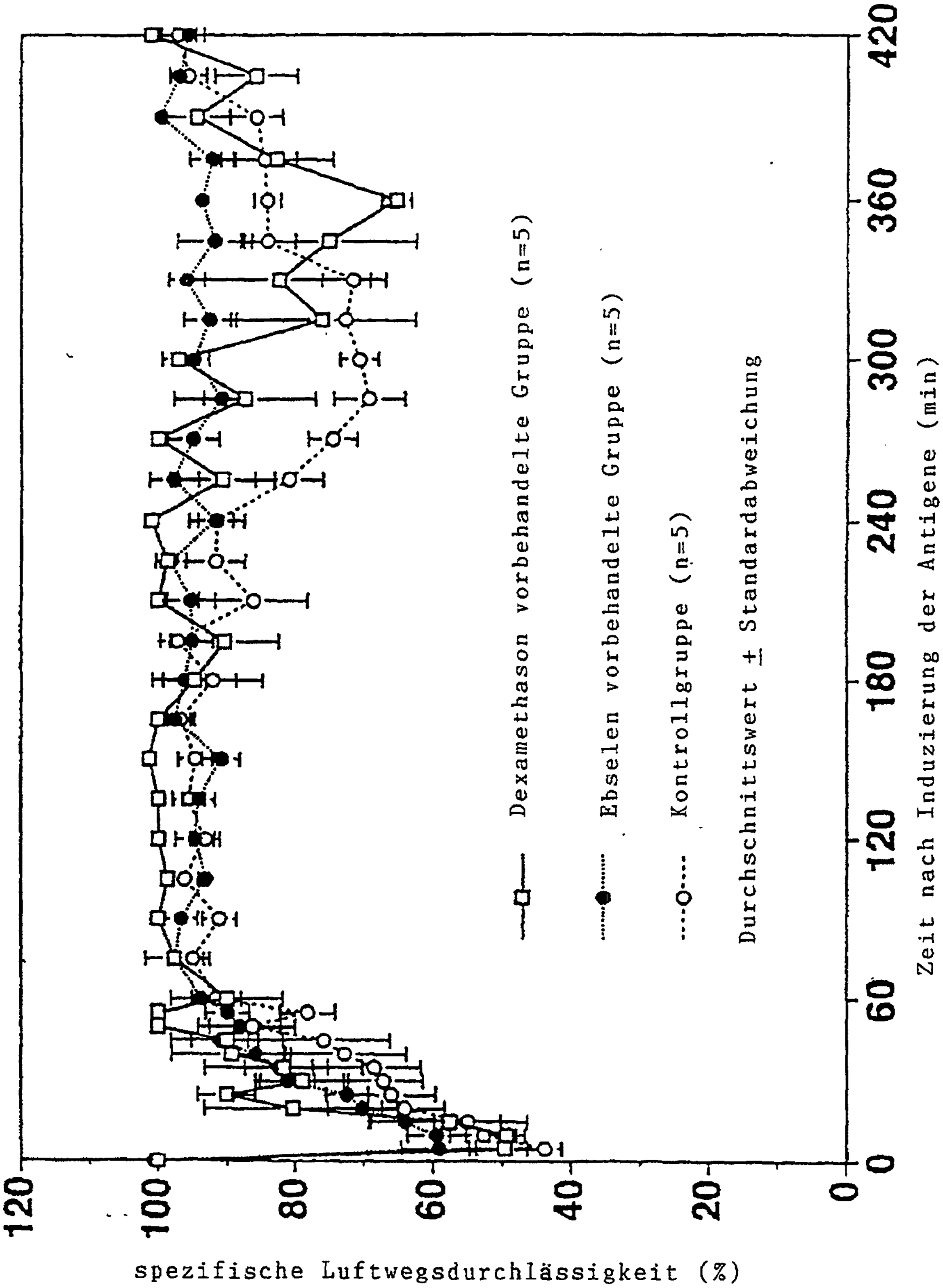
Dexamethason vorbehandelte Gruppe (n=5)
Ebselen vorbehandelte Gruppe (n=5)
Kontrollgruppe (n=5)
Durchschnittswert ± Standardabweichung
0
60
120
180
240
300
360
420
Zeit nach Induzierung der Antigene (min)
120
100
80
60
40
20
0
spezifische Luftwegsdurchlässigkeit (%)